# EUROPEAN PATENT SPECIFICATION

(11) **EP 0 882 085 B1**
(45) Date of publication and mention of the grant of the patent: **24.07.2002**
(21) Application number: 97906686.7
(22) Date of filing: 19.02.1997
(51) Int. Cl.: C08G 67/04, A61L 28/00, A61K 9/20, A61K 9/00

(54) **BIODEGRADABLE POLYMER NETWORKS FOR USE IN ORTHOPEDIC AND DENTAL APPLICATIONS**
BIOLOGISCH ABBAUBARES VERNETZTES POLYMER FÜR ORTHOPÄDISCHE UND ZAHNÄRTZLICHE ZWECKE
RESEAUX DE POLYMERES BIODEGRADABLES DESTINES A DES APPLICATIONS ORTHOPEDIQUES ET DENTAIRES

(30) Priority: 20.02.1996 US 603171
(43) Date of publication of application: 09.12.1998
(73) Proprietor: Massachusetts Institute Of Technology, Cambridge, MA 02142-1493 (US)
(72) Inventor: ANSETH, Kristi S., Boulder, Colorado 80301 (US); LANGER, Robert, S., Newton, MA 02158 (US); SHASTRI, Venkatram R., Allston, MA 02134 (US)
(74) Representative: Bassett, Richard Simon
(86) International application number: US9702618
(87) International publication number: WO9730104

(56) References cited:
- EP-A- 0 266 603
- WO-A-89/00855
- WO-A-89/01006
- US-A- 5 236 563

## Description

### Background of the Invention

The present invention relates generally to methods for crosslinking anhydride monomers or oligomers to form biodegradable, crosslinked polymer networks for use in orthopedic and dental applications.

This invention was sponsored in part by the National Institutes of Health through a fellowship to Kristi S. Anseth and NIH Grant No. AR41972. The government has certain rights in the invention.

A variety of different orthopedic and dental implants have been developed. Metallic orthopedic devices, have been fabricated, however these devices shield stress during healing and can lead to bone atrophy. Hanafusa *et al., Clin. Ortho. Rel. Res*., 315:261 (1995). Poly(methyl methacrylate) (PMMA) is a polymer which is widely used in current bone cement systems. The material is molded prior to implantation as it is polymerizing which allows a limited "window" of processing time. While the physical and mechanical properties of PMMA are appropriate for load-bearing applications the material is non-degradable which can hinder healing.

The manufacture of absorbable orthopaedic devices from a variety of different materials has been described, *e.g.*: sutures and surgical elements made from polyglycolide (U.S. Pat. No. 3,297,033 and U.S. Pat. No. 3,739,773) sutures from polylactide (U.S. Pat. No. 2,703,316), sutures from glycolide/lactide copolymers (U.S. Pat. No. 3,839,297), sutures and osteosynthesis devices from poly-β-hydroxybutyric acid (G.B. Pat. No. 1 034 123), sutures and osteosynthesis devices from polydioxanone (U.S. Pat. No. 4,052,988), and surgical devices from polyesteramides (U.S. Pat. No. 4,343,931). These devices typically are plates which are fixed to bone by screws, cylindrical nails, or corresponding structures manufactured by melting, molding, or pressing the polymer into the desired form. Typically, the tensile strengths of the unhydrolyzed samples are between 40-80 MPa which is modest compared to the strength of cortical bone (about 80 to 200 MPa). In addition, many of these systems are either too brittle or flexible to be used in many bone surgical applications. Thus, the existing applications of resorbable polymers in orthopaedic surgery have been limited because of difficulties associated with processing and the physicomechanical properties of the polymers.

Degradable polyesters such poly (L-lactic acid), poly(glycolic acid), and poly(lactic-co-glycolic acid) are approved for human use by the Food and Drug Administration, and have been used in many medical applications, for example, in sutures. These polymers, however, lack many properties necessary for restoring function in high load-bearing bone applications, since they undergo homogeneous, bulk degradation which is detrimental to the long-term mechanical properties of the material and leads to a large burst of acid products near the end of degradation. In contrast, surface eroding polymers (such as polyanhydrides) maintain their mechanical integrity by preserving the molecular weight of the polymer and exhibit a gradual loss in size which permits bone ingrowth. However, current linear polyanhydride systems have limited mechanical strength.

Photopolymerizable systems have been developed for use in dentistry. Anseth *et al., Adv. Polym. Sci*, 122:177 (1995); U.S. Patent No. 4,872,936 to Engelbrecht; and U.S. Patent No. 5,367,002. In dentistry, methacrylate-based resins are photocured to produce restorative materials, however, these materials are nondegradable and permanent. Synthetic photopolymerizable systems also have been used in opthamology (*e.g.,* U.S. Patent No. 4,919,151 to Grubbs *et al.*). Synthetic photopolymerizable systems have been developed to replace the lens in the eye, after cataract formation, consisting of a polyether with urethane linkages end-capped with acrylate, methacrylate, or styrene. As in the dental applications, the photopolymerized polymer is a permanent and nondegrading system. Photopolymerizable systems have also been used in adhesion prevention (Hill-West *et al., Obstet. Gynecol.,* 83:59 (1994)). In adhesion prevention, degradable and photopolymerizable hydrophilic oligomers of poly(ethylene glycol) end-capped with lactic acid and acrylate functionalities have been developed. While degradable, these systems are hydrogels of limited mechanical strength and degrade on a relatively short timescale.

There is a need for biodegradable polymers which can be used in dental and orthopedic applications. There also is a need for methods for forming polymeric implants which provide mechanical strength but which also are biodegradable *in vivo.* There further is a need for biodegradable polymers which can be polymerized *in vivo,* and which can be readily implanted and shaped for a particular application.

It is therefore an object of the present invention to develop biodegradable polymers which have optimal mechanical properties, particularly as the polymer degrades, for orthopaedic applications. It is a further object of the invention to develop crosslinkable prepolymers which rapidly polymerize at a room temperature, which can be used to form polymerized biodegradable implants with varying different geometries. It is still another object of the invention to provide biodegradable polymers for use in dental and orthopedic applications which biodegrade *in vivo* at a rate which can be determined by the composition and degree of crosslinking of the polymer.

### Summary of the Invention

Biodegradable polymer networks are provided which are useful in a variety of dental and orthopedic applications. The biodegradable polymer networks can be formed in one embodiment by polymerizing anhydride prepolymers including crosslinkable groups, such as unsaturated moieties. The anhydride prepolymers can be crosslinked, for example in a photopolymerization reaction by irradiation of the prepolymer with light in the presence of a free radical initiator. Suitable anhydride prepolymers include dianhydrides of a dicarboxylic acid and a carboxylic acid molecule comprising a crosslinkable group. For example, methacrylic acid dianhydrides of monomers or oligomers of a diacid such as sebacic acid or 1,5-bis(p-carboxyphenoxy)-hexane can be used. In one embodiment, the anhydride prepolymers can be applied *in vivo* to a site where an orthopedic implant is needed, and then may be crosslinked, for example, by irradiation with ultraviolet light, to form a biodegradable implant. The implants advantageously provide mechanical support and also are capable of slowing surface degradation to permit bone ingrowth.

### Detailed Description of the Invention

Crosslinkable anhydride monomers or oligomers are provided which are capable of reacting to form highly crosslinked, biodegradable polyanhydride networks, which are useful in a variety of different biomedical applications. In one embodiment, the anhydride monomers or oligomers may be crosslinked by radiation-induced polymerization, for example, by photopolymerization. The high degree of crosslinking produces polymers with enhanced mechanical properties. The crosslinked polymers are capable of surface controlled degradation under *in vivo* conditions. The rate of degradation can be controlled by selection of the polymer network composition and the crosslinking density within the polymer network. In one embodiment, the crosslinked polymers can be formed *in vivo* by the photopolymerization of anhydride monomers or oligomers, and may be designed and shaped as required for a variety of different orthopedic and dental applications.

### Crosslinkable Anhydride Monomers and Oligomers

Biodegradable crosslinked polymer networks are formed by crosslinking functionalized anhydride monomers or oligomers. Useful functionalized monomers or oligomers include mixed anhydrides of a diacid and a carboxylic acid molecule which includes a crosslinkable group such as an unsaturated moiety. Exemplary anhydride monomers or oligomers include mixed anhydrides of diacids, such as sebacic acid or 1,6-bis(p-carboxyphenoxy)-hexane (MCPH), and a carboxylic acid including an unsaturated moiety such as methacrylic acid. The functionalized anhydride monomers or oligomers are formed, for example, by reacting the diacid with an activated form of the acid, such as an anhydride thereof, to form a mixed anhydride.

Other dicarboxylic acids, or multifunctional acids, or mixtures thereof, can be used, such as dodecanedioic acid, fumaric acid, bis(p-carboxyphenoxy)methane, 1,6-bis(p-carboxyphenoxy)propane, terephthalic acid, isophthalic acid, p-carboxyphenoxy acetic acid, p-carboxyphenoxy valeric acid, p-carboxyphenoxy octanoic acid, or citric acid, which are capable of being functionalized by forming mixed anhydrides with a carboxylic acid comprising a crosslinkable group. The carboxylic acid molecule including a crosslinkable group, in the functionalized prepolymer, can be, for example, a carboxylic acid including an unsaturated moiety, such as methacrylic acid, or other functionalized carboxylic acids, including, *e.g.*, acrylic, methacrylic, vinyl and/or styryl groups.

Preferably, the crosslinkable groups are photopolymerizable groups, such as alkenes, which may be polymerized in a free radical reaction upon irradiation with light in the presence of an initiator. Crosslinkable groups include, for example, acrylates, diacrylates, oligoacrylates, methacrylates, dimethacrylates, oligomethoacrylates, or other biologically acceptable photopolymerizable groups. The functionalized anhydride prepolymers thus in one embodiment may be in the form of a linear anhydride with a crosslinkable unsaturated moiety at each terminus of the linear anhydride.

The resulting prepolymers, consisting of functionalized anhydride monomers and oligomers, including crosslinkable groups such as unsaturated moieties, can be crosslinked, for example, in a photopolymerization reaction to produce highly crosslinked biodegradable polymer networks. The hydrolyzable anhydride linkages make the material biodegradable, and the rate of degradation readily can be controlled by changes in the network composition and the crosslinking density.

As used herein the term "biodegradable" refers to the ability of a material to degrade in the body by being broken down by processes such as hydrolysis or metabolic degradation.

### Crosslinking of Functionalized Monomers

An example of the synthesis of the mixed anhydride functionalized monomers, and the subsequent crosslinking of the monomers to form a crosslinked polymer network is shown in Scheme I. In this embodiment, functionalized monomers (and oligomers) of sebacic acid (MSA) and 1,6-bis(p-carboxyphenoxy)-hexane (MCPH) were synthesized and polymerized. First the dicarboxylic acid monomers were converted to their mixed anhydride with methacrylic anhydride by heating at reflux. 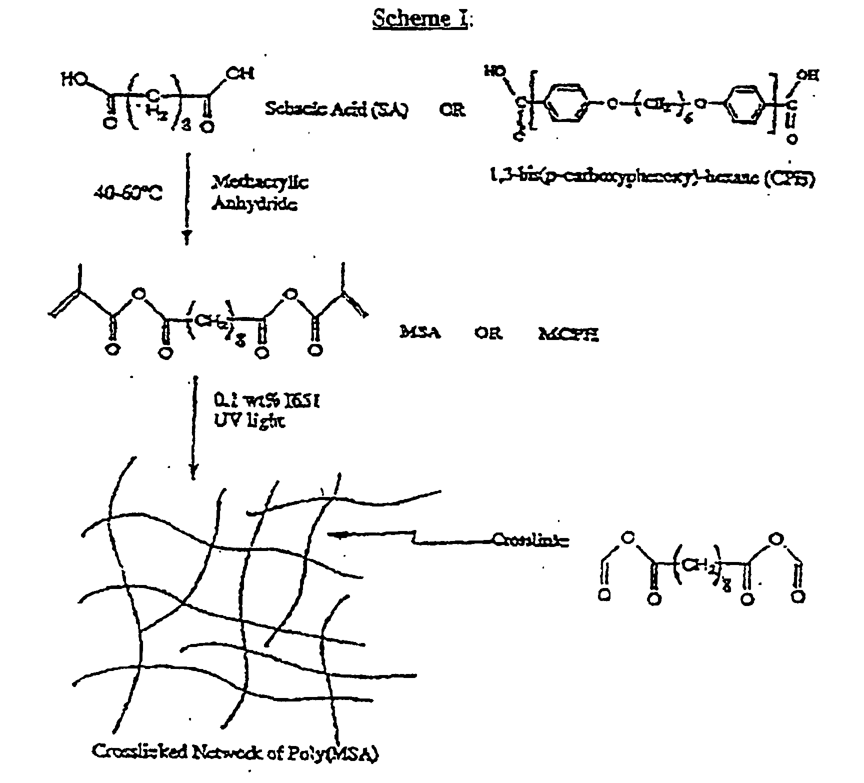 The functionalized monomeric or oligomeric prepolymers were isolated and purified by vacuum distillation or by dissolving in methylene chloride and precipitation from diethyl ether.

In the illustrative embodiment shown in Scheme I, photopolymerization was initiated with 0.1 wt % of 2,2-dimethoxy-2-phenylacetophenone dissolved in each monomer and ultraviolet light of varying intensity (0.1 mW/cm² to 1000 mW/cm²). The high concentration of double bonds in the system and the multifunctional nature of the monomer (two double bonds per monomer molecules) causes the formation of a highly crosslinked polymer system in a period of a few seconds, depending on the initiation rate.

Fourier-transform infrared spectroscopy (FTIR) and differential scanning photocalorimetry (DPC) can be used to characterize the polymerization behavior, curing time, and maximum double bond conversion in these systems. In systems for orthopedic applications, both the polymerization time and maximum conversion are critical factors with desirable systems polymerizing in less than one minute and approaching 100% conversion of their functional groups.

The crosslinking density and the hydrophilicity of the crosslinked biodegradable polymers may be altered by copolymerizing the diacid, such as MSA and MCPH, in various proportions. For example, MSA may be used to increase the hydrophilicity of the resulting network, while MCPH may be used to increase the hydrophobicity. The polymerization conditions, including polymer composition and crosslinking density, polymerization time, and light intensity, may be optimized for a particular application. In particular, the monomers and reagents can be selected in order to optimize the degradation characteristics of the polymer and the material strength.

The mechanical properties of the highly crosslinked materials are significantly improved in the tensile modulus as compared to existing degradable materials. The crosslinkable systems provide not only great flexibility in processing, but also enhanced mechanical properties of the resulting polymer.

### Crosslinking Agents

In a preferred embodiment, crosslinking groups are crosslinked by radiation-induced polymerization, for example, by irradiation with light. In one embodiment, the crosslinking groups can be crosslinked by irradiation with ultraviolet or visible light at a wavelength of between about 250 and 700 nm. For example, ultraviolet light at a wavelength of about 365 nm, or red visible light at a wavelength of about 642 nm, or blue visible light at a wavelength of between 470 and 490 nm may be used. The use of blue light at a wavelength of between 470 and 490 nm is useful for dental applications. Additionally, electron beams, x-rays, γ-rays and other forms of radiation may be used to initiate polymerization of prepolymers.

Biocompatible photoinitiators can be used to initiate free radical polymerization of the prepolymers within a short time frame, minutes at most and most preferably seconds. Exemplary photoinitiators include Irgacure 651™ (2,2-dimethoxy-2-phenylacetophenone), dyes such as eosin dye, and initiators such as 2,2-dimethyl-2-phenylacetophenone, 2-methoxy-2-phenylacetophenone, and camphorquinon. Other free radical initiators include, *e.g.*, an α-diketone, a tertiary amine, a tertiary phosphine, an organic peroxide, peroxides in combination with a reducing agent, aliphatic and aromatic ketones, benzoin, benzoin ethers, benzil and benzil ketals, or combinations thereof. For example, benzophenone or acetophone can be used. Using such initiators, prepolymers may be polymerized *in situ*, for example, by long wavelength ultraviolet light or by focused laser light.

Thermal polymerization initiator systems also may be used, which can initiate free radical polymerization at physiological temperatures including, for example, potassium persulfate, benzoylperoxide, and ammonium persulfate. Additionally, ionic initiators, including cationic initiators may be used.

The prepolymers can be applied *in vivo* and then reagents, such as light curing agents, or other initiating systems, such as thermal and redox systems can be applied, or the prepolymers can be combined with crosslinking agents or fillers prior to *in vivo* application.

### In Vivo Applications

The biodegradable crosslinked polymers can be used in a variety of biomedical applications, including musculoskeletal and dental applications. The biodegradable polymers are biocompatible, strong, easily fashioned, and degradable, and can be polymerized *in vivo* to allow easy placement and fabrication. The polymers degrade at a controlled rate depending on the composition and crosslinking of the polymer, eliminating the need for retrieval.

In orthopedic applications, *in situ* polymerization eliminates the need for shaping the implant with blades, burrs, and warming instruments. Additionally, the process provides a faster and better mechanism for fabricating complex geometries and improves adhesion of the polymer implant to the bone. Photoinitiated polymerizations allow spatial control of the polymerization so that complex patterns can be produced using lasers to produce desired shapes. In addition, the materials can be injection molded and reacted as a thermoset to produce desired shapes from molds *ex vivo.* The materials can be used in many applications requiring load-bearing capacities and controlled degradation. In a preferred embodiment, the compression modulus of the polymers is on the order of about 100 MPa to about 20,000 MPa.

The biodegradable networks permit treatment of bone fractures through fixation since the crosslinked polymers provide sufficient strength to permit fixation, good tissue/material compatibility, and facile molding (into potentially complex shapes) for easy placement. In addition, controlled degradation of the polymers permits optimum bone function upon healing. The materials can reestablish the mechanical integrity of the bone and subsequently degrade to allow new bone formation to bear load and remodel. These properties are a major advantage of the degradable polymeric materials over metallic orthopedic devices which shield stress during healing and can lead to bone atrophy. The biodegradable polymer networks disclosed herein, in contrast, are surface eroding polymers (controlled by hydrophobicity and/or crosslinking density) which maintain their mechanical integrity and undergo a gradual loss in size which permits bone ingrowth.

In the embodiment wherein photopolymerizable functionalized anhydride monomers or oligomers are used, the use of photoinitiation to crosslink the prepolymers greatly simplifies the clinical insertion of orthopaedic polymer implants. For example, in pin applications, a viscous, liquid monomer may be introduced into a pin hole and the system photopolymerized *in situ* to render a hardened polymer of the required dimensions. Photopolymerizable systems are beneficial for many reasons including fast curing rates at room temperature, spatial control of the polymerization, and complete ease of fashioning and flexibility during implantation. The use of photopolymerizable orthopaedic implants provides a broad range of systems which can be designed for a particular surgical application. Degradable polymeric implants also eliminate the need for implant retrieval and can be used simultaneously to deliver therapeutic drugs.

The anhydride prepolymers can be applied to the site in the body of an animal where an implant is needed and then polymerized, or may be polymerized prior to *in vivo* application, to provide shaped implants within the body which can serve a mechanical function, including, without limitation, rods, pins, screws, and plates. The prepolymers and/or initiating agents can be provided in combination with a pharmaceutically acceptable carrier for implantation.

The prepolymers can be combined with fillers, reinforcement materials, radioimaging materials, excipients or other materials as needed for a particular implant application. Examples of fillers include calcium-sodium-metaphosphate which is described in U.S. Patent No. 5,108,755, the disclosure of which is incorporated herein by reference.

### Drug Delivery

The polymerizable functionalized anhydride monomers and/or oligomers optionally can be provided in combination with other degradable or nondegradable polymers, fillers and/or drugs, either before or after polymerization.

The crosslinked biodegradable polymers may be used to deliver therapeutic or diagnostic agents *in vivo.* Examples of drugs which can be incorporated into the prepolymers and in the resulting crosslinked polymers include proteins, carbohydrates, nucleic acids, and inorganic and organic biologically active molecules. Specific examples include enzymes, antibiotics, antineoplastic agents, local anesthetics, hormones, antiangiogenic agents, antibodies, neurotransmitters, psychoactive drugs, drugs affecting reproductive organs, and oligonucleotides such as antisense oligonucleotides. In orthopedic applications, bone regenerating molecules, seeding cells, and/or tissue can be incorporated into the prepolymer prior to or after polymerization, or may be applied prior to or after formation of the implant at the site of implantation. For example bone morphogenic proteins such as those described in U.S. Patent No. 5,011,691, the disclosure of which is incorporated herein by reference, can be used in these applications.

The present invention will be further understood by reference to the following non-limiting examples. In the examples, the following materials and methods were used.

### Materials

Sebacic acid (SA) and methacrylic anhydride (MA) were used as received from Aldrich, and 1,6-bis(carboxyphenoxy) hexane (CPH) was synthesized as described in Conix, *Macromol. Synth.*, 2:95 (1966). Photopolymerizations were initiated with 0.1 wt% Irgacure® 651 (I651, Ciba Geigy).

### Methods

Infrared spectroscopy (Nicolet Magna 550 FTIR), ¹H NMR (Nicolet 360 MHz), and gel permeation chromatography (Perkin-Elmer, isocratic LC pump 250, oven 101, and LC-30 RI detector at 254 nm) were used to characterize reaction products during the functionalization of the diacid monomers. Differential scanning photocalorimetry (Perkin-Elmer DSC7) and infrared spectroscopy were used to monitor the cure behavior of the functionalized monomers. Samples were polymerized with an ultraviolet-visible light curing system (EFOS, Ultracure (100SS) at varying light intensities. Mechanical properties of the resulting polymers were measured using a dynamic mechanical analyzer (Perkin-Elmer, DMA7) and degradation rates were characterized by mass loss.

### Example 1: Preparation and Photopolymerization of Monomers

Functionalized monomers (and oligomers) of sebacic acid (MSA) and 1,6-bis(p-carboxyphenoxy)-hexane (MCPH) were synthesized and subsequently polymerized as illustrated in Scheme I. First the dicarboxylic acid monomers were converted to their mixed anhydride with methacrylic anhydride by heating at reflux. The functionalized monomer was isolated and purified by vacuum distillation, or by dissolving in methylene chloride and precipitation from diethyl ether. Photopolymerizations were initiated with 0.1 wt% of I651 dissolved in each monomer and ultraviolet light of varying intensity (0.1 mW/cm² to 1000 mW/cm²). The high concentration of double bonds in the system and the multifunctional nature of the monomer (two double bonds per monomer molecules) led to the formation of a highly crosslinked polymer system in a period of a few seconds, depending on the initiation rate.

Both Fourier-transform infrared spectroscopy (FTIR) and differential scanning photocalorimetry (DPC) were used to characterize the polymerization behavior, curing time, and maximum double bond conversion in these systems. In the FTIR spectrum for the polymerization of MSA with 0.1 wt% 1651 and approximately 50 mW/cm² of UV light, the methacrylate double bond exhibited a sharp and distinct absorbance near 1640 cm⁻¹ from which the total double bond conversion could be calculated. After 10 seconds of exposure, the system reached nearly 45% conversion of its functional groups. With continued irradiation, the double bond absorbance further decreased until a maximum double bond conversion of 94% was reached. Attainment of a maximum double bond conversion in highly crosslinked polymers has been previously reported (Ruyter and Oysaed, *CRC Crit. Rev. Biocomp*, 4:247 (1988)) and results from severe restrictions on the mobility of the reacting species. Thus, these systems are useful in orthopedic applications, where both the polymerization time and maximum conversion are critical factors, and wherein desirable systems polymerize less than 1 minute and approach 100% conversion of their functional groups.

In addition, the crosslinking density and the hydrophilicity of the polymer were altered by copolymerizing MSA and MCPH in various proportions. MSA was used to increase the hydrophilicity of the resulting network while MCPH increased the hydrophobicity. The polymerization method (including copolymer composition and crosslinking density, polymerization time, and light intensity) can be readily optimized for a particular application, to optimize the degradation characteristics of the polymer and the material strength.

### Example 2: Evaluation of the Mechanical Properties of the Crosslinked Biodegradable Polymers.

The highly crosslinked materials made as described in Example 1 had significant improvements in the tensile modulus as compared to existing degradable materials. Table 1 provides a comparison of mechanical properties of bone (Yaszemski, Ph.D. Thesis, Massachusetts Institute of Technology, 1995) with that of the crosslinked polyanhydrides.

**Table 1:**

| **Comparison of Material Properties.** | | | |
|---|---|---|---|
| **Material** | **Modulus** | | |
| | **Longitudinal** | **Compression** | **Shear** |
| Cortical Bone | 17-20 GPa | 17-20 GPa | 3 GPa |
| Trabecular Bone | 50-100 MPa* | 50-100 MPa | |
| poly(MSA) | 1 GPa | | 2 GPa |
| poly(MCPH) | 500 MPa | | 900 MPa |

| | | | |
|---|---|---|---|
| *depends strongly on the density which varies from 0.1 to 1.0 g/cm³ | | | |

There was a significant increase in the modulus of functionalized and crosslinked poly(MCPH) (500 MPa) as compared to linear CPH (1.3 MPa). Leong *et al., J. Biomed. Mater. Res.,* 19:941 (1985). Other approaches to increasing the mechanical strength of linear polyanhydrides have focused on incorporating imide groups into the polymer backbone. The most promising materials in this class have shown compression strengths of 36-56 MPa (Uhrich *et al., Macromolecules* 28:2184 (1995)), but do not approach the strengths seen with the crosslinked materials. Finally, resorbable sutures of poly(lactic acid) have initial compression yield stresses of 50-60 MPa, but the efficacy in many orthopedic applications is further limited by bulk degradation that occurs on a relatively short time interval, compared to the polyanhydrides. Pulapura and Kohn, *J*. *Biomater. Appl.*, 6:216 (1992). Thus, the photopolymerizable and crosslinkable systems provide not only great flexibility in processing, but also enhanced mechanical properties of the resulting polymer.

## Claims

1. A method for making biodegradable polymer networks comprising:
a) providing anhydride prepolymers which comprise mixed anhydrides of
i) a monomer or oligomer of a diacid or multifunctional acid and
ii) a carboxylic acid molecule which includes a crosslinkable group,
wherein the crosslinkable group is an unsaturated moiety and wherein the prepolymers are linear with a crosslinkable group at each terminus: and
b) crosslinking the anhydride prepolymers, to form a crosslinked biodegradable polymer network.

2. The method of claim 1 wherein the crosslinkable groups comprise unsaturated moieties selected from the group consisting of acrylic, methacrylic, vinyl and styryl.

3. The method of claim 2 wherein, in step b), the anhydride prepolymers are crosslinked by a photopolymerization.

4. The method of claim 3 wherein the photopolymerization is initiated by irradiation of the prepolymers with light in the presence of an initiator, such as an α-diketone, a tertiary amine, a tertiary phosphine, an organic peroxide, peroxides in combination with a reducing agent, aliphatic ketones, aromatic ketones, benzoin, benzoin ethers, benzil, benzil ketals, and combinations thereof.

5. The method of claim 4 wherein the prepolymers are irradiated with ultraviolet light.

6. The method of claim 1 wherein the anhydride prepolymer comprises a dianhydride of a dicarboxylic acid and a carboxylic acid molecule comprising a crosslinkable group.

7. The method of claim 6 wherein the crosslinkable group comprises an unsaturated group selected from the group consisting of acrylic, methacrylic, vinyl and styryl.

8. The method of claim 6 wherein the dicarboxylic acid comprises a dicarboxylic acid mixture.

9. The method of claim 6 wherein the anhydride prepolymer comprises a methacrylic acid dianhydride of a monomer or oligomer of a diacid selected from the group consisting of sebacic acid and 1,6-bis(p-carboxyphenoxy)-hexane.

10. The use of anhydride prepolymers comprising mixed anhydrides of
i) a monomer or oligomer of a diacid or multifunctional acid and
ii) a carboxylic acid molecule which includes a crosslinkable group,
wherein the crosslinkable group is an unsaturated moiety and wherein the prepolymers are linear with a crosslinkable group at each terminus for use in the manufacture of a medicament used in forming an implant in vivo, in a method comprising:
a) application of the medicament to a site in the body of a mammal where an implant is needed and
b) crosslinking the anhydride prepolymers, to form a crosslinked biodegradable polymer network in the form of an implant at the site.

11. The use of claim 10 modified according to any one of claims 2, 3, 4, 5, 6, 7 or 9.

12. The use of claim 10, modified according to claim 4 and wherein the initiator is applied in combination with the prepolymers in step a).

13. The use of claim 10 wherein the implant is selected from the group consisting of an orthopedic and a dental implant.

14. The use of claim 10 wherein the implant comprises an orthopedic implant selected from the group consisting of rods, pins, screws, and plates.

15. A method for forming a biodegradable implant comprising photopolymerizing anhydride prepolymers comprising mixed anhydrides of
i) a monomer or oligomer of a diacid or multifunctional acid and
ii) a carboxylic acid molecule which includes a crosslinkable group.
wherein the crosslinkable group is an unsaturated moiety and wherein the prepolymers are linear with a crosslinkable group at each terminus, to form a crosslinked biodegradable polymer network in the form of an implant.

16. The method of claim 15 modified according to claim 6 or 9.

17. A composition for forming a biocompatible, biodegradable polymeric implant, the composition comprising anhydride prepolymers in combination with a pharmaceutically acceptable carrier, wherein the anhydride prepolymers comprise mixed anhydrides of
i) a monomer or oligomer of a diacid or multifunctional acid and
ii) a carboxylic acid molecule which includes a crosslinkable group,
wherein the crosslinkable group is an unsaturated moiety and wherein the prepolymers are linear with a crosslinkable group at each terminus.

18. The composition of claim 17 wherein the crosslinkable groups comprise an unsaturated moiety selected from the group consisting of acrylic, methacrylic, vinyl and styryl.

19. The composition of claim 18 wherein the composition further comprises an initiator selected from the group consisting of a free radical initiator and an ionic initiator.

20. The composition of claim 19 wherein the initiator is selected from the group consisting of an α-diketone, a tertiary amine, a tertiary phosphine, an organic peroxide, peroxides in combination with a reducing agent, aliphatic ketones, aromatic ketones, benzoin, benzoin ethers, benzil, benzil ketals, and combinations thereof.

21. The composition of claim 17 wherein the anhydride prepolymer comprises a dianhydride of a dicarboxylic acid monomer or oligomer and a carboxylic acid molecule comprising an unsaturated moiety.

22. The composition of claim 21 wherein the anhydride prepolymer comprises a methacrylic acid dianhydride of a monomer or oligomer of a diacid selected from the group consisting of sebacic acid and 1,6-bis(p-carboxyphenoxy)-hexane.

23. The composition of claim 17 further comprising a therapeutic agent or a diagnostic agent.

24. A biodegradable implant comprising a crosslinked biodegradable polymer network formed by crosslinking anhydride prepolymers, wherein the anhydride prepolymers comprise mixed anhydrides of
i) a monomer or oligomer of a diacid or multifunctional acid and
ii) a carboxylic acid molecule which includes a crosslinkable group,
wherein the crosslinkable group is an unsaturated moiety and wherein the prepolymers are linear with a crosslinkable group at each terminus.

25. The biodegradable implant of claim 24 its formation being modified according to any one of claims 2, 6 or 9.

26. The method of claim 6, wherein the dicarboxylic acid is selected from the group consisting of sebacic acid, dodecanedioic acid, fumaric acid, bis(p-carboxyphenoxy)methane,
1,6-bis(p-carboxyphenoxy)propane, terephthalic acid, isophthalic acid,
p-carboxyphenoxy acetic acid, p-carboxyphenoxy valeric acid,
p-carboxyphenoxy octanoic acid, and citric acid.

## Patentansprüche

1. Verfahren zur Herstellung biologisch abbaubarer Polymer-Netzwerke, das umfasst:
a) Bereitstellen von Anhydrid-Präpolymeren, die gemischte Anhydride sind aus
i) einem Monomer oder Oligomer einer Disäure oder multifunktionellen Säure und
ii) einem Carbonsäuremolekül, das eine vernetzbare Gruppe aufweist,
wobei die vernetzbare Gruppe eine ungesättigte Gruppe ist und wobei die Präpolymere linear sind und an beiden Enden eine vernetzbare Gruppe aufweisen; und
b) Vernetzen der Anhydrid-Präpolymere zur Bildung eines vernetzten biologisch abbaubaren Polymer-Netzwerks.

2. Verfahren nach Anspruch 1, wobei die vernetzbaren Gruppen ungesättigte Gruppen umfassen, die aus der Gruppe ausgewählt sind, die aus Acryl, Methacryl, Vinyl und Styryl besteht.

3. Verfahren nach Anspruch 2, wobei im Schritt b die Anhydrid-Präpolymere durch Photopolymerisation vernetzt werden.

4. Verfahren nach Anspruch 3, wobei die Photopolymerisation durch Bestrahlung der Präpolymere mit Licht in Gegenwart eines Initiators initiiert wird, beispielsweise eines α-Diketons, eines tertiären Amins, eines tertiären Phosphins, eines organischen Peroxids, von Peroxiden in Kombination mit einem Reduktionsmittel, von aliphatischen Ketonen, aromatischen Ketonen, Benzoin, Benzoinethern, Benzil, Benzilketalen und Kombinationen von diesen.

5. Verfahren nach Anspruch 4, wobei die Präpolymere mit ultraviolettem Licht bestrahlt werden.

6. Verfahren nach Anspruch 1, wobei das Anhydrid-Präpolymer ein Dianhydrid einer Dicarbonsäure und eines Carbonsäuremoleküls, das eine vernetzbare Gruppe aufweist, umfasst.

7. Verfahren nach Anspruch 6, wobei die vernetzbare Gruppe eine ungesättigte Gruppe umfasst, die aus der Gruppe ausgewählt ist, die aus Acryl, Methacryl, Vinyl und Styryl besteht.

8. Verfahren nach Anspruch 6, wobei die Dicarbonsäure eine Mischung von Dicarbonsäuren umfasst.

9. Verfahren nach Anspruch 6, wobei das Anhydrid-Präpolymer ein Methacrylsäure-Dianhydrid eines Monomers oder Oligomers einer Disäure umfasst, die aus der Gruppe ausgewählt ist, die aus Sebacinsäure und 1,6-Bis(p-carboxyphenoxy)hexan besteht.

10. Verwendung von Anhydrid-Polymeren, die gemischte Anhydride umfassen aus
i) einem Monomer oder Oligomer einer Disäure oder multifunktionellen Säure und
ii) einem Carbonsäuremolekül, das eine vernetzbare Gruppe aufweist,
wobei die vernetzbare Gruppe eine ungesättigte Gruppe ist und wobei die Präpolymere linear sind und an beiden Enden eine vernetzbare Gruppe aufweisen, für die Verwendung bei der Herstellung eines Medikaments, das bei der Bildung eines Implantats in vivo verwendet wird, in einem Verfahren, das umfasst:
a) Einbringen des Medikaments in eine Stelle im Körper eines Säugetiers, an der ein Implantat benötigt wird, und
b) Vernetzen der Anhydrid-Präpolymere unter Bildung eines vernetzten biologisch abbaubaren Polymer-Netzwerks in der Form eines Implantats an dieser Stelle.

11. Verwendung von Anspruch 10, der gemäß einem beliebigen der Ansprüche 2, 3, 4, 5, 6, 7 oder 9 modifiziert ist.

12. Verwendung von Anspruch 10, der gemäß Anspruch 4 modifiziert ist und wobei der Initiator zusammen mit den Präpolymeren in Schritt a eingesetzt wird.

13. Verwendung von Anspruch 10, wobei das Implantat aus der Gruppe ausgewählt ist, die aus einem orthopädischen Implantat und einem Dentalimplantat besteht.

14. Verwendung von Anspruch 10, wobei das Implantat ein orthopädisches Implantat umfasst, das aus der Gruppe ausgewählt ist, die aus Stäben, Zapfen, Schrauben und Platten besteht.

15. Verfahren zur Herstellung eines biologisch abbaubaren Implantats, das die Photopolymerisierung von Anhydrid-Präpolymeren umfasst, die gemischte Anhydride umfassen von:
i) einem Monomer oder Oligomer einer Disäure oder multifunktionellen Säure und
ii) einem Carbonsäuremolekül, das eine vernetzbare Gruppe aufweist,
wobei die vernetzbare Gruppe eine ungesättigte Gruppe ist und wobei die Präpolymere linear sind und an beiden Enden eine vernetzbare Gruppe aufweisen, zur Erzeugung eines vernetzten biologisch abbaubaren Polymer-Netzwerks in der Form eines Implantats.

16. Verfahren nach Anspruch 15, das gemäß dem Anspruch 6 oder 9 modifiziert ist

17. Zusammensetzung für die Herstellung eines biokompatiblen, biologisch abbaubaren Polymerimplantats, wobei die Zusammensetzung Anhydrid-Präpolymere in Kombination mit einem pharmazeutisch annehmbaren Träger umfasst, wobei die Anhydrid-Präpolymere gemischte Anhydride umfassen von:
i) einem Monomer oder Oligomer einer Disäure oder multifunktionellen Säure und
ii) einem Carbonsäuremolekül, das eine vernetzbare Gruppe aufweist,
wobei die vernetzbare Gruppe eine ungesättigte Gruppe ist und wobei die Präpolymere linear sind und an beiden Enden eine vernetzbare Gruppe aufweisen.

18. Zusammensetzung nach Anspruch 17, wobei die vernetzbaren Gruppen ungesättigte Gruppen sind, die aus der Gruppe ausgewählt sind, die aus Acryl, Methacryl, Vinyl und Styryl besteht.

19. Zusammensetzung nach Anspruch 18, wobei die Zusammensetzung außerdem einen Initiator aufweist, der aus der Gruppe ausgewählt ist, die aus einem Initiator in Form eines freien Radikals oder eines ionischen Initiators besteht.

20. Zusammensetzung nach Anspruch 19, wobei der Initiator aus der Gruppe ausgewählt ist, die besteht aus einem α-Diketon, einem tertiären Amin, einem tertiären Phosphin, einem organischen Peroxid, Peroxiden in Kombination mit einem Reduktionsmittel, aliphatischen Ketonen, aromatischen Ketonen, Benzoin, Benzoinethern, Benzil, Benzilketalen und Kombinationen von diesen.

21. Zusammensetzung nach Anspruch 17, wobei das Anhydrid-Präpolymer ein Dianhydrid eines Dicarbonsäuremonomers oder -oligomers und eines Carbonsäuremoleküls, das eine vernetzbare Gruppe aufweist, umfasst.

22. Zusammensetzung nach Anspruch 21, wobei das Anhydrid-Präpolymer ein Methacrylsäure-Dianhydrid eines Monomers oder Oligomers einer Disäure umfasst, die aus der Gruppe ausgewählt ist, die aus Sebacinsäure und 1,6-Bis(p-carboxy-phenoxy)hexan besteht.

23. Zusammensetzung nach Anspruch 17, die außerdem ein therapeutisches Mittel oder ein diagnostisches Mittel aufweist.

24. Biologisch abbaubares Implantat, das ein vernetztes biologisch abbaubares Polymer-Netzwerk umfasst, das durch das Vernetzen von Anhydrid-Präpolymeren gebildet wird,
wobei die Anhydrid-Präpolymere gemischte Anhydride umfassen aus
i) einem Monomer oder Oligomer einer Disäure oder multifunktionellen Säure und
ii) einem Carbonsäuremolekül, das eine vernetzbare Gruppe aufweist,
wobei die vernetzbare Gruppe eine ungesättigte Gruppe ist und wobei die Präpolymere linear sind und an beiden Enden eine vernetzbare Gruppe aufweisen.

25. Biologisch abbaubares Implantat nach Anspruch 24, wobei seine Herstellung gemäß einem beliebigen der Ansprüche 2, 6 oder 9 modifiziert ist.

26. Verfahren nach Anspruch 6, wobei die Dicarbonsäure aus der Gruppe ausgewählt ist, die besteht aus Sebacinsäure, Dodecandisäure, Fumarsäure, Bis(p-carboxyphenoxy)methan, 1,6-Bis(p-carboxyphenoxy)propan, Terephthalsäure, Isophthalsäure, p-Carboxyphenoxy-essigsäure, p-Carboxyphenoxyvaleriansäure, p-Carboxyphenoxyoctansäure und Zitronensäure.

## Revendications

1. Procédé de fabrication de réseaux polymères biodégradables comprenant :
a) la fourniture de prépolymères d'anhydrides qui comprennent un mélange d'anhydrides de :
i) un monomère ou oligomère d'un diacide ou d'un acide multifonctionnel et,
ii) une molécule d'acide carboxylique qui comprend un groupement réticulable, dans lequel le groupement réticulable est un fragment insaturé et dans lequel les prépolymères sont linéaires avec un groupement réticulable à chaque extrémité et,
b) la réticulation des prépolymères d'anhydrides pour former un réseau polymère réticulé biodégradable.

2. Procédé selon la revendication 1 dans lequel les groupements réticulables comprennent des fragments insaturés choisis dans le groupe constitué par les radicaux acrylique, méthacrylique, vinyle et styryle.

3. Procédé selon la revendication 2 dans lequel à l'étape b) les prépolymères d'anhydrides sont réticulés par photopolymérisation.

4. Procédé selon la revendication 3 dans lequel la photopolymérisation est amorcée par irradiation des prépolymères avec de la lumière en présence d'un amorceur, tel qu'une α-dicétone, une amine tertiaire, une phosphine tertiaire, un peroxyde organique, les peroxydes en combinaison avec un agent réducteur, les cétones aliphatiques, les cétones aromatiques, la benzoïne, les éthers de benzoïne, le benzile, les acétals de benzile et les combinaisons de ceux-ci.

5. Procédé selon la revendication 4 dans lequel les prépolymères sont irradiés par une lumière ultraviolette.

6. Procédé selon la revendication 1 dans lequel le prépolymère d'anhydrides comprend un dianhydride d'un acide dicarboxylique et une molécule d'acide carboxylique comprenant un groupement réticulable.

7. Procédé selon la revendication 6 dans lequel le groupement réticulable comprend un groupement insaturé choisi dans le groupe constitué par un radical acrylique, méthacrylique, vinyle et styryle.

8. Procédé selon la revendication 6 dans lequel l'acide dicarboxylique comprend un mélange d'acides dicarboxyliques.

9. Procédé selon la revendication 6 dans lequel le prépolymère d'anhydrides comprend un dianhydride d'acide méthacrylique d'un monomère ou oligomère d'un diacide choisi dans le groupe constitué par l'acide sébacique et le 1,6-bis(p-carboxyphénoxy)-hexane.

10. Utilisation de prépolymères d'anhydrides comprenant des mélanges d'anhydrides de :
i) un monomère ou oligomère d'un diacide ou d'un acide multifonctionnel et,
ii) une molécule d'acide carboxylique qui comprend un groupement réticulable, dans lequel le groupement réticulable est un fragment insaturé et dans lequel les prépolymères sont linéaires avec un groupement réticulable à chaque extrémité,
pour l'utilisation dans la fabrication d'un médicament utilisé dans la formation d'un implant in vivo, dans un procédé comprenant :
a) l'application du médicament sur un site du corps d'un mammifère où un implant est nécessaire et,
b) la réticulation des prépolymères d'anhydrides pour former un réseau polymère réticulé biodégradable sous la forme d'un implant au site.

11. Utilisation selon la revendication 10 modifiée selon l'une quelconque des revendications 2, 3, 4, 5, 6, 7 ou 9.

12. Utilisation selon la revendication 10, modifiée selon la revendication 4 et dans laquelle l'amorceur est appliqué en combinaison avec les prépolymères dans l'étape a).

13. Utilisation selon la revendication 10 dans laquelle l'implant est choisi dans le groupe constitué par un implant orthopédique et un implant dentaire.

14. Utilisation selon la revendication 10 dans laquelle l'implant comprend un implant orthopédique choisi dans le groupe constitué par les tiges, les broches, les vis et les plaques.

15. Procédé de formation d'un implant biodégradable comprenant la photopolymérisation de prépolymères d'anhydrides comprenant un mélange d'anhydrides de :
i) un monomère ou oligomère d'un diacide ou d'un acide multifonctionnel et,
ii) une molécule d'acide carboxylique qui comprend un groupement réticulable, dans lequel le groupement réticulable est un fragment insaturé et dans lequel les prépolymères sont linéaires avec un groupement réticulable à chaque extrémité, pour former un réseau polymère réticulé biodégradable sous la forme d'un implant.

16. Procédé selon la revendication 15, modifié selon la revendication 6 ou 9.

17. Composition pour former un implant polymère biocompatible et biodégradable, la composition comprenant des prépolymères d'anhydrides en combinaison avec un support pharmaceutiquement acceptable, dans laquelle les prépolymères d'anhydrides comprennent un mélange d'anhydrides de :
i) un monomère ou oligomère d'un diacide ou acide multifonctionnel et,
ii) une molécule d'acide carboxylique comprenant un groupement réticulable, dans lequel le groupement réticulable est un fragment insaturé et dans lequel les prépolymères sont linéaires avec un groupement réticulable à chaque extrémité.

18. Composition selon la revendication 17 dans laquelle les groupements réticulables comprennent un fragment insaturé choisi dans le groupe constitué par les radicaux acrylique, méthacrylique, vinyle et styryle.

19. Composition selon la revendication 18 dans laquelle la composition comprend également un amorceur choisi dans le groupe constitué par un amorceur de radicaux libres et un amorceur ionique.

20. Composition selon la revendication 19 dans laquelle l'amorceur est choisi dans le groupe constitué par une α-dicétone, une amine tertiaire, une phosphine tertiaire, un peroxyde organique, les peroxydes en combinaison avec un agent réducteur, les cétones aliphatiques, les cétones aromatiques, la benzoïne, les êthers de benzoïne, le benzile, les acétals de benzile et les combinaisons de ceux-ci.

21. Composition selon la revendication 17 dans laquelle le prépolymère d'anhydrides comprend un dianhydride d'un monomère ou oligomère d'acide dicarboxylique et une molécule d'acide carboxylique comprenant un fragment insaturé.

22. Composition selon la revendication 21 dans laquelle le prépolymère d'anhydride comprend un dianhydride d'acide méthacrylique d'un monomère ou oligomère d'un diacide choisi dans le groupe constitué par l'acide sébacique et le 1,6-bis(p-carboxyphénoxy)-hexane.

23. Composition selon la revendication 17 comprenant également un agent thérapeutique ou un agent diagnostique.

24. Implant biodégradable comprenant un réseau polymère réticulé biodégradable formé par réticulation de prépolymères d'anhydrides, dans lequel les prépolymères d'anhydrides comprennent des mélanges d'anhydrides de :
i) un monomère ou oligomère d'un diacide ou d'un acide multifonctionnel et,
ii) une molécule d'un acide carboxylique qui comprend un groupement réticulable, dans lequel le groupement réticulable est un fragment insaturé et dans lequel les prépolymères sont linéaires et comprennent un groupement réticulable à chaque extrémité.

25. Implant biodégradable selon la revendication 24, sa formation étant modifiée selon l'une quelconque des revendications 2, 6, ou 9.

26. Procédé selon la revendication 6 dans lequel l'acide dicarboxylique est choisi dans le groupe constitué par l'acide sébacique, l'acide dodécanoïque, l'acide fumarique, bis(p-carboxyphénoxy)méthane, le 1,6-bis(p-carboxyphénoxy)propane, l'acide téréphtalique, l'acide isophtalique, l'acide p-carboxyphénoxyacétique, l'acide p-carboxyphénoxyvalérique, l'acide p-carboxyphénoxyoctanoïque et l'acide citrique.
